# EUROPEAN PATENT APPLICATION

(11) **EP 4 667 014 A1**
(43) Date of publication of application: **24.12.2025**
(21) Application number: 24757102.9
(22) Date of filing: 06.02.2024
(51) Int. Cl.: A61K 38/46, A61P 25/28, A61P 25/24, A23L 33/17

(54) **COMPOSITION FOR PREVENTING OR TREATING NEURODEGENERATIVE DISEASES OR DEPRESSION, COMPRISING ASM PROTEIN OR FRAGMENT THEREOF**

(30) Priority: 14.02.2023 KR 20230019496
(71) Applicant: Kyungpook National University Industry-Academic Cooperation Foundation, Daegu 41566 (KR)
(72) Inventor: BAE, Jae Sung, Daegu 41566 (KR); JIN, Hee Kyung, Daegu 41566 (KR); PARK, Min Hee, Daegu 41566 (KR); CHOI, Byung Jo, Daegu 41566 (KR)
(74) Representative: JWP Patent & Trademark Attorneys
(86) International application number: PCT/KR2024/001708
(87) International publication number: WO 2024/172373

(57) **Abstract**

The present invention relates to a composition for preventing or treating neurodegenerative diseases or depression, comprising ASM protein or a fragment thereof and, more particularly, to a pharmaceutical or food composition comprising ASM protein or a fragment thereof that exhibits a therapeutic effect on neurodegenerative diseases or depression by inhibiting the activity of ASM protein in plasma.

## Description

### [Technical Field]

The present application claims priority to Korean Patent Application No. 10-2023-0019496, filed on February 14, 2023, and the entirety of the specification thereof is incorporated herein by reference.

The present disclosure relates to a composition for preventing or treating neurodegenerative diseases or depression comprising ASM protein or a fragment thereof, and more specifically, to a pharmaceutical or food composition comprising ASM protein or a fragment thereof, which exhibits therapeutic effects on neurodegenerative diseases or depression by inhibiting the activity of ASM protein in plasma.

### [Background of the Invention]

Sphingolipid metabolism regulates normal cellular signal transduction, and abnormal changes in sphingolipid metabolism affect various neurodegenerative diseases, including Alzheimer's disease. Meanwhile, ASM (acid sphingomyelinase), an enzyme that regulates sphingolipid metabolism, is a protein expressed in almost all types of cells and plays an important role in sphingolipid metabolism and cell membrane turnover.

In the brains of patients with neurodegenerative diseases, including Alzheimer's disease, the expression of ASM is significantly increased compared to normal individuals, and it has been reported that inhibiting the overexpression of ASM or inhibiting the activity of ASM suppresses the accumulation of amyloid-β (Aβ), improves learning and memory, and exhibits therapeutic effects on neurodegenerative diseases (Korean Patent No. 10-1521117). Additionally, recent reports indicate that the activity of ASM is increased in neurological disorders such as depression, and the inhibition of ASM shows depression-relieving effects (Nature Medicine. 2013 Jul 19(7):934-938, PLoS One. 2016 Sep 6;11(9):e0162498). Therefore, the development of ASM inhibitors, that is, substances capable of inhibiting the expression or activity of ASM, is promising as a useful therapeutic method for various diseases caused by the increase of ASM, including neurodegenerative diseases and depression.

Meanwhile, although direct ASM inhibitors have not yet been developed, several indirect inhibitors of ASM have been identified. First, tricyclic antidepressants (e.g., amitriptyline (AMI), desipramine, imipramine, etc.), which are the most widely used indirect ASM inhibitors, are antidepressant drugs that are actually used in clinical practice. Although they were not initially developed as ASM inhibitors, various research results have proven that these drugs exhibit ASM inhibitory effects. The main mode of action of tricyclic antidepressants is the enhancement of neurotransmitter activity through the inhibition of neurotransmitter reuptake in nerve cells, and they exhibit ASM inhibitory effects as a secondary action. However, in the case of tricyclic antidepressants, as they act on the nervous system and nerve cells, they may cause side effects such as drowsiness, increased light sensitivity, and vomiting. Therefore, there is a need to develop new drugs that can directly inhibit ASM activity.

### [Problem to be Solved]

In general, for proteins whose expression is increased in specific diseases, the increase in protein expression is closely associated with the pathological mechanism, and inhibiting this is often recognized as a method of treating the disease. In the case of ASM protein, it has been reported that the expression of ASM is significantly increased in the brains of patients with neurodegenerative diseases, including Alzheimer's disease, compared to normal individuals, and that inhibiting the overexpression of ASM or its activity suppresses the accumulation of amyloid-β (Aβ), improves learning and memory, and exhibits therapeutic effects on neurodegenerative diseases. Nevertheless, the inventors of the present disclosure discovered that administering ASM protein to animal models of neurodegenerative diseases, such as Alzheimer's disease, where the activity of ASM protein is increased, rather reduces the activity of ASM protein, and consequently improves neurodegenerative diseases, thereby completing the present invention.

Accordingly, an object of the present disclosure is to provide a pharmaceutical composition for preventing or treating neurodegenerative diseases or depression comprising ASM (acid sphingomyelinase) protein or a fragment thereof; a vector comprising a polynucleotide encoding ASM (acid sphingomyelinase) protein or a fragment thereof; or a cell comprising the vector as an active ingredient.

Another object of the present disclosure is to provide a pharmaceutical composition for preventing or treating neurodegenerative diseases or depression consisting of ASM (acid sphingomyelinase) protein or a fragment thereof; a vector comprising a polynucleotide encoding ASM (acid sphingomyelinase) protein or a fragment thereof; or a cell comprising the vector.

Another object of the present disclosure is to provide a pharmaceutical composition for preventing or treating neurodegenerative diseases or depression consisting essentially of ASM (acid sphingomyelinase) protein or a fragment thereof; a vector comprising a polynucleotide encoding ASM (acid sphingomyelinase) protein or a fragment thereof; or a cell comprising the vector.

Another object of the present disclosure is to provide a food composition for preventing or improving neurodegenerative diseases or depression comprising ASM (acid sphingomyelinase) protein or a fragment thereof; a vector comprising a polynucleotide encoding ASM (acid sphingomyelinase) protein or a fragment thereof; or a cell comprising the vector as an active ingredient.

Another object of the present disclosure is to provide a food composition for preventing or improving neurodegenerative diseases or depression consisting of ASM (acid sphingomyelinase) protein or a fragment thereof; a vector comprising a polynucleotide encoding ASM (acid sphingomyelinase) protein or a fragment thereof; or a cell comprising the vector.

Another object of the present disclosure is to provide a food composition for preventing or improving neurodegenerative diseases or depression consisting essentially of ASM (acid sphingomyelinase) protein or a fragment thereof; a vector comprising a polynucleotide encoding ASM (acid sphingomyelinase) protein or a fragment thereof; or a cell comprising the vector.

Another object of the present disclosure is to provide the use of ASM (acid sphingomyelinase) protein or a fragment thereof; a vector comprising a polynucleotide encoding ASM (acid sphingomyelinase) protein or a fragment thereof; or a cell comprising the vector for preparing a composition for treating neurodegenerative diseases or depression.

Another object of the present disclosure is to provide a method for treating neurodegenerative diseases or depression comprising administering to a subject in need thereof a composition comprising ASM (acid sphingomyelinase) protein or a fragment thereof; a vector comprising a polynucleotide encoding ASM (acid sphingomyelinase) protein or a fragment thereof; or a cell comprising the vector as an active ingredient.

### [Means for Solving the Problem]

To achieve the aforementioned objectives of the present disclosure, the present disclosure provides a pharmaceutical composition for preventing or treating neurodegenerative diseases or depression comprising ASM (acid sphingomyelinase) protein or a fragment thereof; a vector comprising a polynucleotide encoding ASM (acid sphingomyelinase) protein or a fragment thereof; or a cell comprising the vector as an active ingredient.

Additionally, the present disclosure provides a pharmaceutical composition for preventing or treating neurodegenerative diseases or depression consisting of ASM (acid sphingomyelinase) protein or a fragment thereof; a vector comprising a polynucleotide encoding ASM (acid sphingomyelinase) protein or a fragment thereof; or a cell comprising the vector.

Furthermore, the present disclosure provides a pharmaceutical composition for preventing or treating neurodegenerative diseases or depression consisting essentially of ASM (acid sphingomyelinase) protein or a fragment thereof; a vector comprising a polynucleotide encoding ASM (acid sphingomyelinase) protein or a fragment thereof; or a cell comprising the vector.

To achieve another objective of the present disclosure, the present disclosure provides a food composition for preventing or improving neurodegenerative diseases or depression comprising ASM (acid sphingomyelinase) protein or a fragment thereof; a vector comprising a polynucleotide encoding ASM (acid sphingomyelinase) protein or a fragment thereof; or a cell comprising the vector as an active ingredient.

Additionally, the present disclosure provides a food composition for preventing or improving neurodegenerative diseases or depression consisting of ASM (acid sphingomyelinase) protein or a fragment thereof; a vector comprising a polynucleotide encoding ASM (acid sphingomyelinase) protein or a fragment thereof; or a cell comprising the vector.

Furthermore, the present disclosure provides a food composition for preventing or improving neurodegenerative diseases or depression consisting essentially of ASM (acid sphingomyelinase) protein or a fragment thereof; a vector comprising a polynucleotide encoding ASM (acid sphingomyelinase) protein or a fragment thereof; or a cell comprising the vector.

To achieve another objective of the present disclosure, the present disclosure provides the use of ASM (acid sphingomyelinase) protein or a fragment thereof; a vector comprising a polynucleotide encoding ASM (acid sphingomyelinase) protein or a fragment thereof; or a cell comprising the vector for preparing a composition for treating neurodegenerative diseases or depression.

To achieve another objective of the present disclosure, the present disclosure provides a method for treating neurodegenerative diseases or depression comprising administering to a subject in need thereof a composition comprising ASM (acid sphingomyelinase) protein or a fragment thereof; a vector comprising a polynucleotide encoding ASM (acid sphingomyelinase) protein or a fragment thereof; or a cell comprising the vector as an active ingredient.

Hereinafter, the present disclosure will be described in detail.

The present disclosure provides a pharmaceutical composition for preventing or treating neurodegenerative diseases or depression comprising ASM (acid sphingomyelinase) protein or a fragment thereof; a vector comprising a polynucleotide encoding ASM (acid sphingomyelinase) protein or a fragment thereof; or a cell comprising the vector as an active ingredient.

In the present disclosure, the "ASM (acid sphingomyelinase) protein" refers to an enzyme that is one of the SMase (sphingomyelinase) family regulating sphingolipid metabolism. The ASM protein catalyzes the process of breaking down sphingomyelin into ceramide and phosphorylcholine and can be classified as alkaline, neutral, or acidic depending on the pH at which it exhibits optimal enzymatic activity.

The ASM protein may include all types of ASM proteins known in the art. Specifically, the ASM protein may be derived from mammals, and more specifically, it may be derived from humans, monkeys, rats, or mice. Additionally, the ASM protein may include all amino acid sequences known as ASM proteins in the art. For example, the ASM protein is disclosed under GENEBANK accession No. AAA58377.1, AAA75008.1, etc., and preferably, it may include the amino acid sequence of SEQ ID NO: 1, and most preferably, it may consist of the amino acid sequence of SEQ ID NO: 1.

In the present disclosure, the term "fragment" refers to a portion of the ASM amino acid sequence and means a polypeptide exhibiting 50% or more, preferably 60% or more, more preferably 70% or more, even more preferably 80% or more, and most preferably 90% or more sequence homology with the amino acid sequence of each protein.

In one aspect of the present disclosure, the "fragment" may refer to the saposin domain of the ASM protein, which includes the amino acid sequence of SEQ ID NO: 2, and preferably, it may consist of the amino acid sequence of SEQ ID NO: 2.

In the present disclosure, the ASM protein or its fragment should be interpreted to include its variants or functional equivalents. The protein variants or functional equivalents refer to polypeptides that exhibit substantially the same activity as the ASM protein of the present disclosure, having 60%, preferably 70%, more preferably 80% or more sequence homology with the amino acid sequence of the ASM protein as a result of addition, substitution, or deletion of amino acids. The functional equivalents may include, for example, modified amino acid sequences in which some amino acids of the ASM protein's amino acid sequence are substituted, deleted, or added. The substitution of amino acids may preferably be conservative substitutions, and examples of conservative substitutions of naturally occurring amino acids include the following: aliphatic amino acids (Gly, Ala, Pro), hydrophobic amino acids (Ile, Leu, Val), aromatic amino acids (Phe, Tyr, Trp), acidic amino acids (Asp, Glu), basic amino acids (His, Lys, Arg, Gln, Asn), and sulfur-containing amino acids (Cys, Met). The deletion of amino acids may preferably be located in regions not directly involved in the activity of the ASM protein of the present disclosure. Additionally, the scope of the functional equivalents may include polypeptide derivatives in which some chemical structures of the polypeptide are modified while maintaining the basic framework and physiological activity of the ASM protein. For example, structural modifications to improve the stability, storability, volatility, or solubility of the ASM protein of the present disclosure, as well as "fusion proteins" created by fusion with other proteins while maintaining physiological activity, may be included.

Additionally, in the present disclosure, the ASM protein may be used in its native form or in the form of a pharmaceutically acceptable salt. In the present disclosure, "pharmaceutically acceptable" means physiologically acceptable and does not inhibit the action of the active ingredient when administered to humans, and does not cause allergic reactions such as gastrointestinal disorders or dizziness, or similar reactions. The salt is preferably an acid addition salt formed by a pharmaceutically acceptable free acid, and the free acid may be an organic acid or an inorganic acid. The organic acid may include, but is not limited to, citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, p-toluenesulfonic acid, glutamic acid, and aspartic acid. The inorganic acid may include, but is not limited to, hydrochloric acid, hydrobromic acid, sulfuric acid, and phosphoric acid.

The ASM protein or its fragment in the present disclosure may be prepared by extraction from natural sources, synthesis, or a gene recombination method based on DNA sequences.

The present disclosure also provides a pharmaceutical composition for preventing or treating neuroinflammatory diseases comprising a vector comprising a polynucleotide encoding ASM protein or its fragment, or a cell comprising the vector as an active ingredient.

The polynucleotide may be DNA or RNA, and the RNA may be mRNA. The nucleotide sequence of the polynucleotide encoding the ASM protein can be easily identified by those skilled in the art through known literature, for example, Gene ID.6609, 20597, etc.

In the present disclosure, the vector may be selected from the group consisting of linear DNA, plasmid DNA, and recombinant viral vectors, and the virus may be selected from the group consisting of retrovirus, adenovirus, adeno-associated virus, herpes simplex virus, and lentivirus.

The cell comprising the vector is preferably selected from the group consisting of hematopoietic stem cells, dendritic cells, autologous tumor cells, and established tumor cells, but is not limited thereto.

The composition of the present disclosure may further include appropriate carriers, excipients, and diluents commonly used in the preparation of pharmaceutical compositions. The pharmaceutical composition according to the present disclosure may be formulated into oral formulations such as powders, granules, tablets, capsules, suspensions, emulsions, syrups, and aerosols, external preparations, suppositories, and sterile injectable solutions according to conventional methods.

Suitable formulations known in the art can be referred to by those skilled in the art.

The carriers, excipients, and diluents that may be included in the pharmaceutical composition of the present disclosure include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, and mineral oil. When formulated, the composition is prepared using diluents or excipients such as fillers, extenders, binders, wetting agents, disintegrants, or surfactants commonly used. Solid formulations for oral administration include tablets, pills, powders, granules, and capsules, and these solid formulations are prepared by mixing at least one excipient, for example, starch, calcium carbonate, sucrose, lactose, or gelatin, with the extract. Additionally, lubricants such as magnesium stearate and talc may be used in addition to simple excipients. Liquid formulations for oral administration include suspensions, internal solutions, emulsions, and syrups, which may include commonly used simple diluents such as water and liquid paraffin, as well as various excipients such as wetting agents, sweeteners, flavoring agents, and preservatives. Formulations for parenteral administration include sterile aqueous solutions, non-aqueous solvents, suspensions, emulsions, freeze-dried formulations, and suppositories. Non-aqueous solvents and suspensions may include propylene glycol, polyethylene glycol, vegetable oils such as olive oil, and injectable esters such as ethyl oleate. Bases for suppositories may include witepsol, macrogol, tween 61, cacao butter, laurin butter, and glycerogelatin.

The term "administration" used in the present disclosure means providing a predetermined composition of the present disclosure to a subject by any appropriate method.

The preferred dosage of the pharmaceutical composition of the present disclosure may vary depending on the condition and body weight of the subject, the severity of the disease, the drug form, the route of administration, and the duration, but can be appropriately selected by those skilled in the art. For a desired effect, the composition of the present disclosure may be administered at a dose of 0.001 to 1000 mg/kg per day. The administration may be performed once a day or divided into several doses. The above dosage does not limit the scope of the present disclosure in any way.

When the pharmaceutical composition of the present disclosure includes a vector comprising a polynucleotide encoding ASM protein or its fragment, it is preferable to include 0.05 to 500 mg, more preferably 0.1 to 300 mg, and in the case of a recombinant virus comprising the polynucleotide encoding the protein, it is preferable to include 10³ to 10¹² IU, but it is not limited thereto.

Additionally, when the pharmaceutical composition of the present disclosure includes cells comprising the polynucleotide encoding the protein, it is preferable to include 10³ to 10⁸ cells, but it is not limited thereto.

The pharmaceutical composition of the present disclosure can be administered to a subject through various routes. All methods of administration can be anticipated, for example, oral, rectal, or intravenous, intramuscular, subcutaneous, intrauterine, epidural, or intracerebrovascular injection.

In the present disclosure, the neurodegenerative diseases may include, without limitation, those in which the activity of ASM protein is increased, for example, dementia, Alzheimer's disease, Parkinson's disease, Huntington's disease, mild cognitive impairment, cerebral amyloid angiopathy, Down syndrome, amyloid stroke, systemic amyloidosis, Dutch-type amyloidosis, Niemann-Pick disease, Pick's disease, multiple sclerosis, Creutzfeldt-Jakob disease, amyotrophic lateral sclerosis, spinocerebellar atrophy, Tourette's syndrome, Friedrich's Ataxia, Machado-Joseph's disease, Lewy body dementia, dystonia, progressive supranuclear palsy, and frontotemporal dementia. Preferably, the neurodegenerative diseases may be mild cognitive impairment, Alzheimer's disease, or dementia, and most preferably, Alzheimer's disease.

The present disclosure also provides a food composition for preventing or improving neurodegenerative diseases or depression comprising ASM (acid sphingomyelinase) protein or a fragment thereof; a vector comprising a polynucleotide encoding ASM (acid sphingomyelinase) protein or a fragment thereof; or a cell comprising the vector as an active ingredient.

There is no particular limitation on the types of food. Examples of foods to which the above substance can be added include drinks, meat, sausages, bread, biscuits, rice cakes, chocolates, candies, snacks, confectionery, pizza, noodles, other types of noodles, gums, ice cream, dairy products including milk, various soups, beverages, alcoholic drinks, vitamin complexes, dairy products, and processed dairy products, and all health functional foods in the conventional sense.

The ASM protein or its fragment of the present disclosure can be added directly to food or used together with other foods or food ingredients and can be appropriately used according to conventional methods. The amount of the active ingredient mixed can be appropriately determined depending on its purpose of use (for prevention or improvement). Generally, the amount of the protein in health foods can be added in an amount of 0.1 to 90 parts by weight based on the total weight of the food. However, for long-term intake for health and hygiene purposes or for health control purposes, the amount may be below the above range, and since there is no problem in terms of safety, the active ingredient can also be used in an amount exceeding the above range.

The health functional beverage composition of the present disclosure does not have any particular limitation on other components except that it contains the protein as an essential component in the indicated ratio, and it may contain various flavoring agents or natural carbohydrates as additional components, like conventional beverages. Examples of the natural carbohydrates mentioned above include monosaccharides such as glucose and fructose; disaccharides such as maltose and sucrose; and polysaccharides such as dextrin and cyclodextrin, as well as sugar alcohols such as xylitol, sorbitol, and erythritol. As flavoring agents other than those mentioned above, natural flavoring agents (thaumatin, stevia extracts such as rebaudioside A, glycyrrhizin, etc.) and synthetic flavoring agents (saccharin, aspartame, etc.) can be advantageously used. The ratio of the natural carbohydrates may generally be about 1 to 20 g, preferably about 5 to 12 g, per 100 g of the composition of the present disclosure.

In addition to the above components, the food composition of the present disclosure may contain various nutrients, vitamins, minerals (electrolytes), synthetic flavoring agents and natural flavoring agents, colorants, and thickeners (cheese, chocolate, etc.), pectic acid and its salts, alginic acid and its salts, organic acids, protective colloid thickeners, pH adjusters, stabilizers, preservatives, glycerin, alcohol, carbonating agents used in carbonated beverages, etc. Furthermore, the food composition of the present disclosure may contain pulp for the preparation of natural fruit juices and fruit juice beverages and vegetable beverages. These components can be used independently or in combination. The ratio of these additives is not limited, but it is generally selected in the range of 0.1 to about 20 parts by weight per 100 parts by weight of the protein of the present disclosure.

The present disclosure also provides the use of ASM (acid sphingomyelinase) protein or a fragment thereof; a vector comprising a polynucleotide encoding ASM (acid sphingomyelinase) protein or a fragment thereof; or a cell comprising the vector for preparing a composition for treating neurodegenerative diseases or depression.

The present disclosure also provides a method for treating neurodegenerative diseases or depression comprising administering to a subject in need thereof a composition comprising ASM (acid sphingomyelinase) protein or a fragment thereof; a vector comprising a polynucleotide encoding ASM (acid sphingomyelinase) protein or a fragment thereof; or a cell comprising the vector as an active ingredient.

The "effective amount" in the present disclosure refers to an amount that, when administered to a subject, exhibits an effect of improving, treating, detecting, diagnosing, or suppressing or reducing neurodegenerative diseases or depression, and the "subject" refers to an animal, preferably a mammal, particularly an animal including humans, and may also include cells, tissues, organs, etc., derived from animals. The subject may be a patient in need of the above effect.

The "treatment" in the present disclosure comprehensively refers to improving symptoms caused by neurodegenerative diseases or depression or the diseases themselves, and it may include curing, substantially preventing, or improving the condition, as well as alleviating, curing, or preventing one or most symptoms caused by the diseases, but is not limited thereto.

The term "comprising" used in this specification is used in the same sense as "including" or "characterized by" and does not exclude additional components or steps not specifically mentioned in the composition or method according to the present disclosure. Additionally, the term "consisting of" means excluding additional elements, steps, or components not specifically described. The term "consisting essentially of" means that the composition or method may include substances or steps that do not substantially affect the basic characteristics of the described substances or steps.

### [Effect of the Invention]

The composition of the present disclosure comprising ASM protein or a fragment thereof as an active ingredient is highly effective in preventing or treating neurodegenerative diseases or depression caused by increased ASM protein activity by inhibiting the activity of ASM protein in the blood.

### [Brief Description of the Drawings]

Fig. 1a and Fig. 1b are diagrams showing ASM activity in the plasma of Alzheimer's patients (Fig. 1a, n=15/group) according to the disease progression stage and in an Alzheimer's animal model (Fig. 1b, n=7∼11/group) according to age (WT: wild type, AD: Alzheimer's animal model (APP/PS1 mouse)).

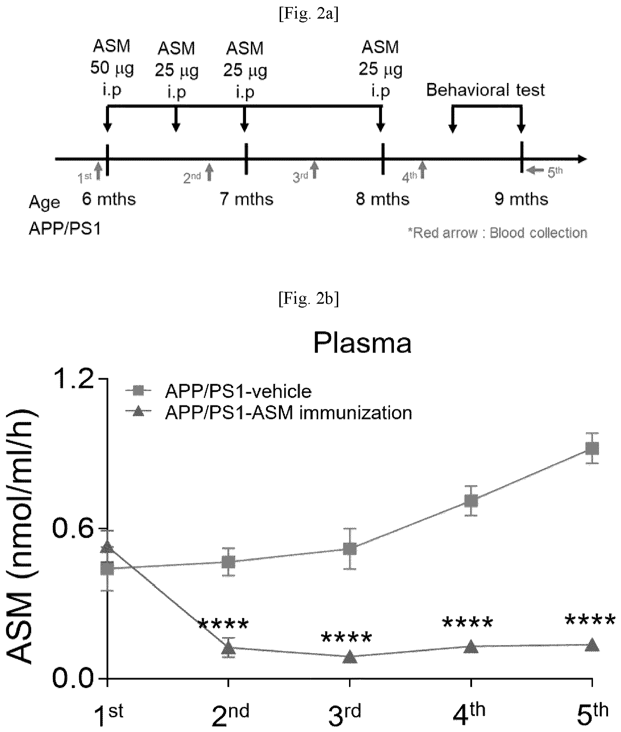

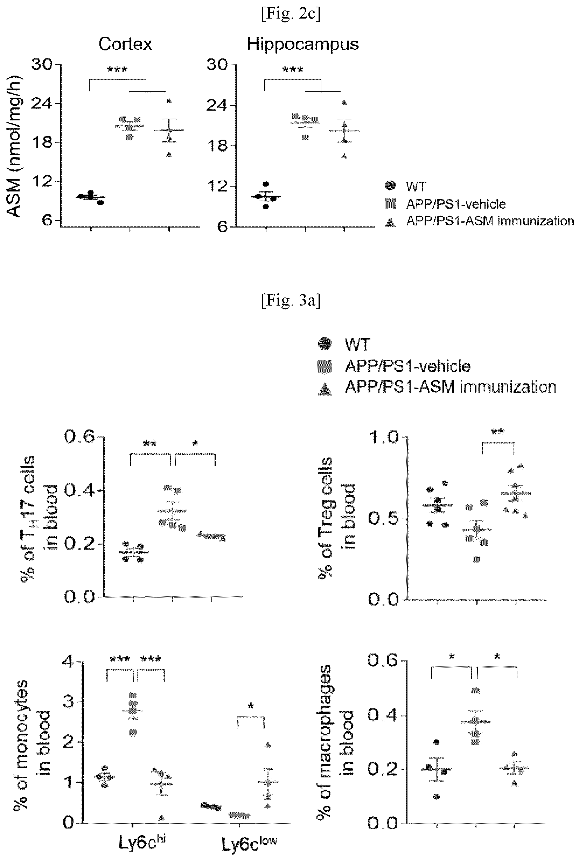

Figs. 2a to 2c are diagrams showing the experimental outline (Fig. 2a) of injecting ASM protein into a 6-month-old Alzheimer's animal model, which is the age before ASM activity increases and before excessive Alzheimer's lesions occur, to investigate the effect of active immunity induced by ASM protein injection on Alzheimer's disease, and the changes in ASM activity in the plasma (Fig. 2b, n=10~15/group) and in the brain cortex and hippocampus (Fig. 2c, n=4/group) of the Alzheimer's animal model after vehicle or ASM protein injection (WT: wild type, AD: Alzheimer's animal model (APP/PS1 mouse)).
Fig. 3a and Fig. 3b are results confirming that the abnormal immune cell composition changes observed in the blood (Fig. 3a) and brain (Fig. 3b) of the Alzheimer's animal model are not observed after ASM protein injection (n=4~7/group) (WT: wild type, AD: Alzheimer's animal model (APP/PS1 mouse)).
Fig. 4a and Fig. 4b are results confirming that excessive amyloid-beta plaque deposition in the brain of the Alzheimer's animal model is prevented by ASM protein injection (WT: wild type, AD: Alzheimer's animal model (APP/PS1 mouse)).

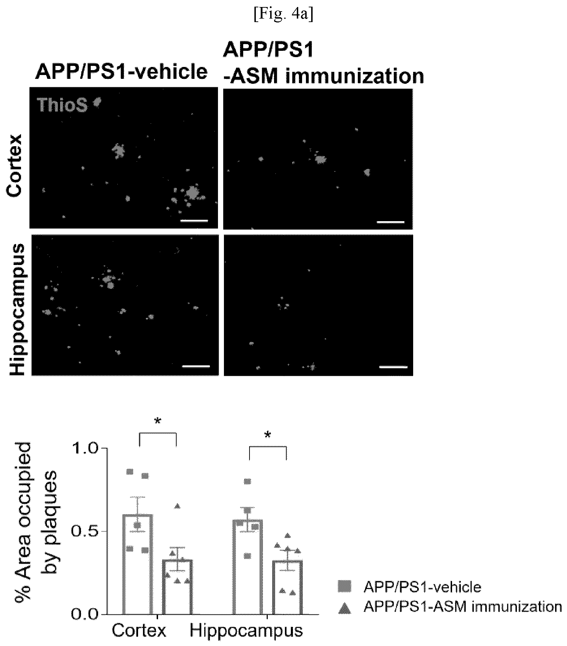

Fig. 4a shows the results of immunofluorescence staining of Thioflavin S (ThioS, fibrillar amyloid-beta plaques) and quantification of the area occupied by fibrillar amyloid-beta plaques in the brain -cortex and hippocampus of the Alzheimer's animal model injected with vehicle or ASM protein (n=5~6/group).

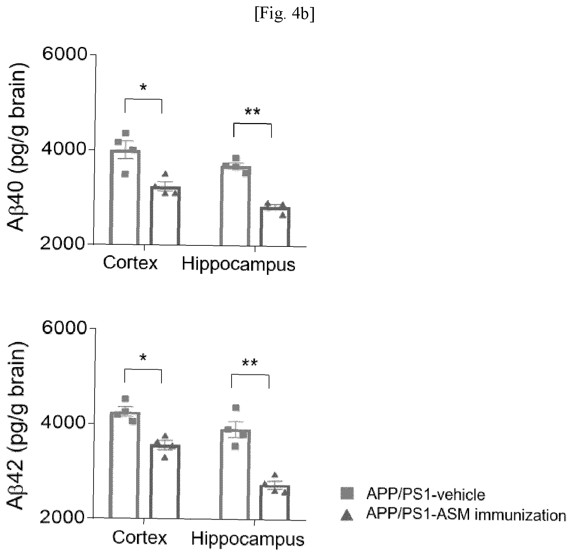

Fig. 4b shows the results of measuring the accumulation of Aβ40 or Aβ42 in the brain cortex and hippocampus of the Alzheimer's animal model injected with vehicle or ASM protein using ELISA (n=4/group).

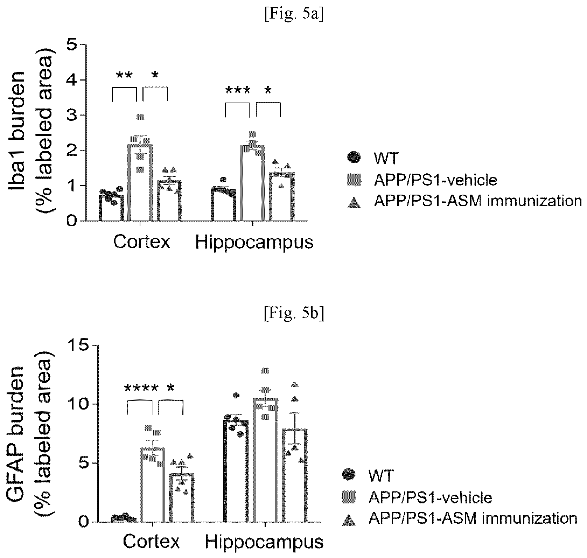

Fig. 5a and Fig. 5b are results confirming that increased neuroinflammation in the brain cortex and hippocampus of the Alzheimer's animal model is not increased by ASM protein injection (WT: wild type, AD: Alzheimer's animal model (APP/PS1 mouse)).
Fig. 5a shows the quantification of the percentage of microglia (Iba-1) in the brain cortex and hippocampus of wild-type mice and Alzheimer's animal models injected with vehicle or ASM protein (n=5/group).
Fig. 5b shows the quantification of the percentage of astrocytes (GFAP) in the brain cortex and hippocampus of wild-type mice and Alzheimer's animal models injected with vehicle or ASM protein (n=5/group).

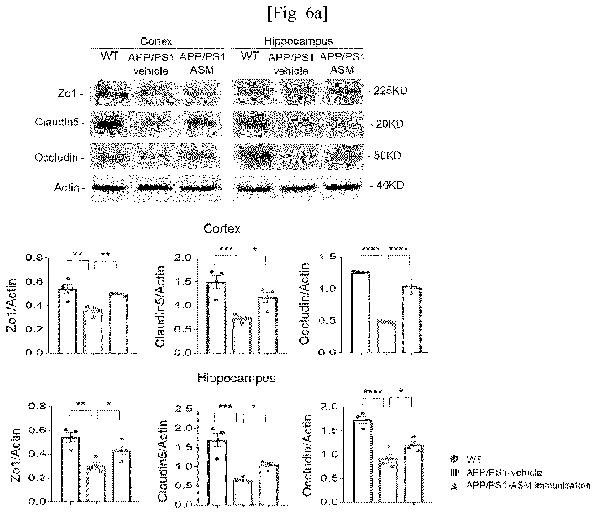

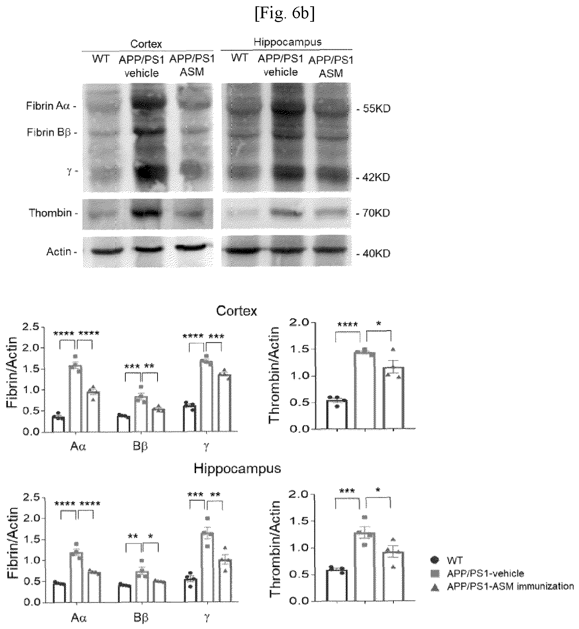

Fig. 6a and Fig. 6b are results confirming that the damaged blood-brain barrier (BBB) tight junction proteins and increased vascular-brain barrier permeability in the brain cortex and hippocampus of the Alzheimer's animal model are prevented by ASM protein injection (WT: wild type, AD: Alzheimer's animal model (APP/PS1 mouse)).
Fig. 6a shows the changes in tight junction proteins Zo1, Claudin5, and Occludin in the brain cortex and hippocampus of wild-type mice and Alzheimer's animal models injected with vehicle or ASM protein (n=4/group).
Fig. 6b shows the changes in the expression of plasma proteins fibrin and thrombin in the brain cortex and hippocampus of wild-type mice and Alzheimer's animal models injected with vehicle or ASM protein (n=4/group).

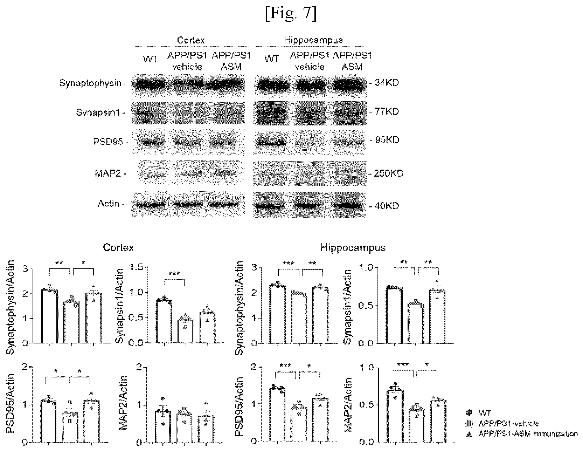

Fig. 7 shows the changes in the expression of synaptic proteins synaptophysin, synapsin1, PSD95, and MAP2 in the brain cortex and hippocampus of wild-type mice and Alzheimer's animal models injected with vehicle or ASM protein (n=4/group) (WT: wild type, AD: Alzheimer's animal model (APP/PS1 mouse)).

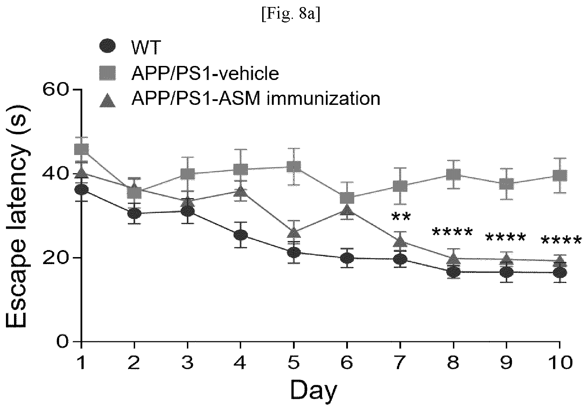

Figs. 8a to 8d are results showing the degree of prevention of learning and cognitive function impairment in Alzheimer's animal models injected with vehicle or ASM protein (WT: wild type, AD: Alzheimer's animal model (APP/PS1 mouse)).
Fig. 8a shows the results of evaluating learning and memory through the Morris water maze test in wild-type mice (n=17), Alzheimer's animal models injected with vehicle (n=14), and Alzheimer's animal models injected with ASM protein (n=20).

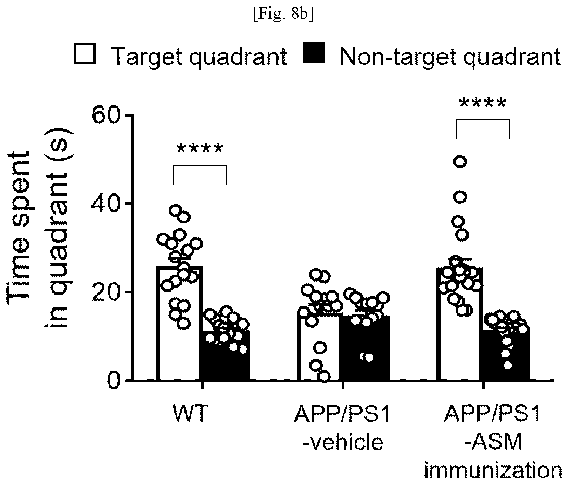

Fig. 8b shows the time spent on the target platform on the 11th day of the test.

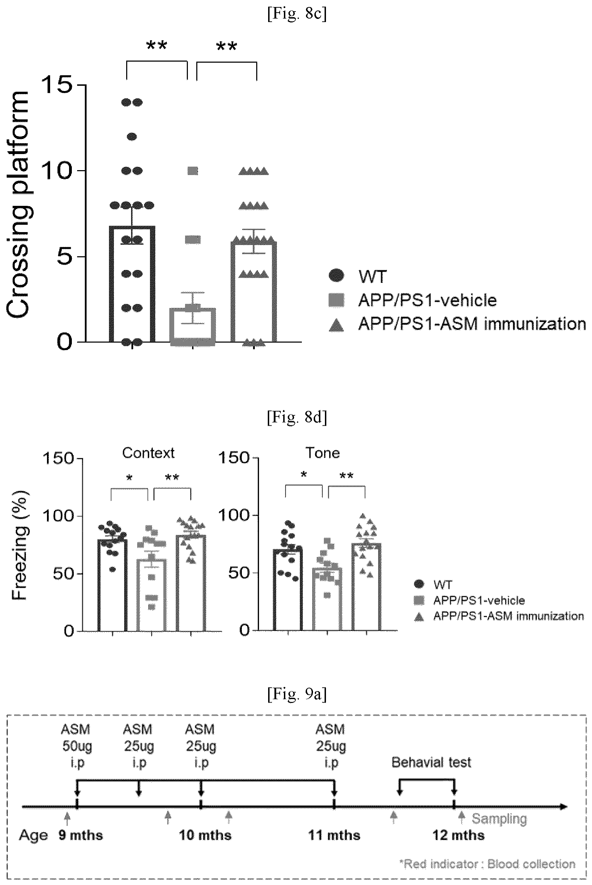

Fig. 8c shows the number of entries into the target area of the platform on the 11th day of the test.
Fig. 8d shows the results of evaluating context and tone memory through the fear conditioning test in wild-type mice (n=14), Alzheimer's animal models injected with vehicle (n=12), and Alzheimer's animal models injected with ASM protein (n=17).

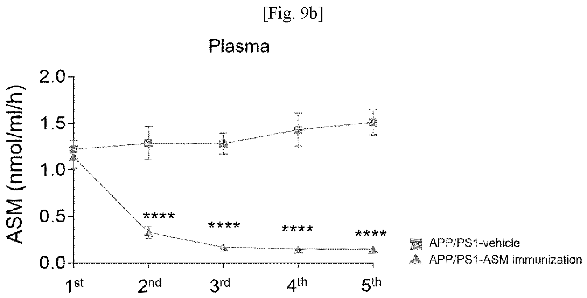

Fig. 9a and Fig. 9b are diagrams showing the experimental outline (Fig. 9a) of injecting ASM protein into a 9-month-old Alzheimer's animal model, which is the age after ASM activity increases, to investigate whether active immunity induced by ASM protein injection affects the improvement of Alzheimer's disease, and the changes in ASM activity in the plasma (Fig. 9b, n=10~12/group) of the Alzheimer's animal model after vehicle or ASM protein injection (AD: Alzheimer's animal model (APP/PS1 mouse)).

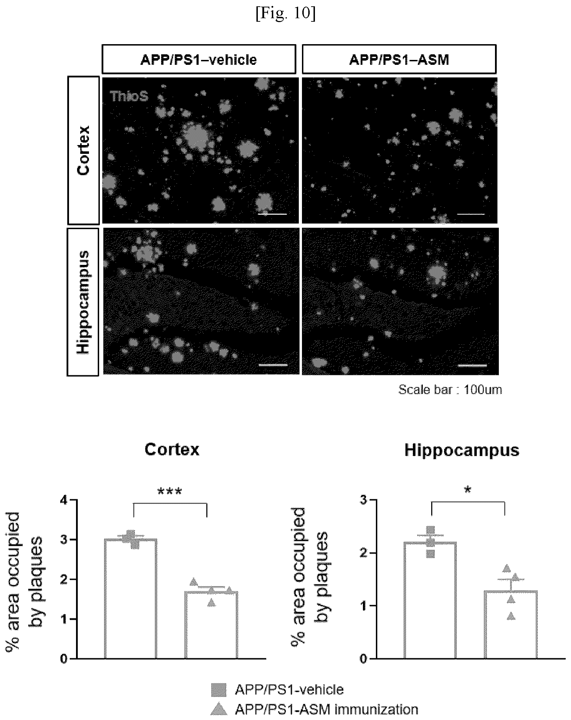

Fig. 10 shows the results of immunofluorescence staining of Thioflavin S (ThioS, fibrillar amyloid-beta plaques) and quantification of the area occupied by fibrillar amyloid-beta plaques in the brain cortex and hippocampus of the Alzheimer's animal model injected with vehicle or ASM protein (n=3~4/group) (AD: Alzheimer's animal model (APP/PS1 mouse)).

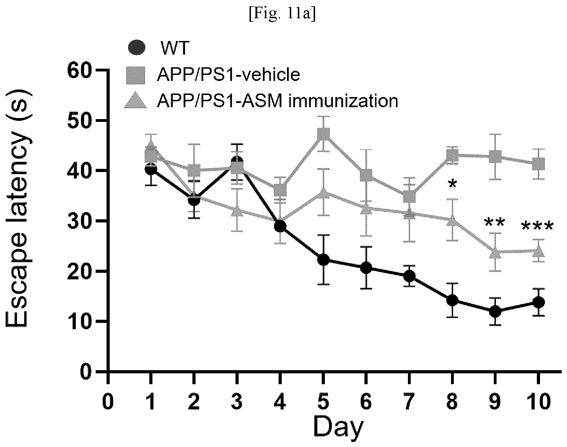

Figs. 11a to 11d are results showing the degree of prevention of learning and cognitive function impairment in 12-month-old Alzheimer's animal models injected with vehicle or ASM protein (WT: wild type, AD: Alzheimer's animal model (APP/PS1 mouse)).
Fig. 11a shows the results of evaluating learning and memory through the Morris water maze test in wild-type mice (n=6), Alzheimer's animal models injected with vehicle (n=6), and Alzheimer's animal models injected with ASM protein (n=6).

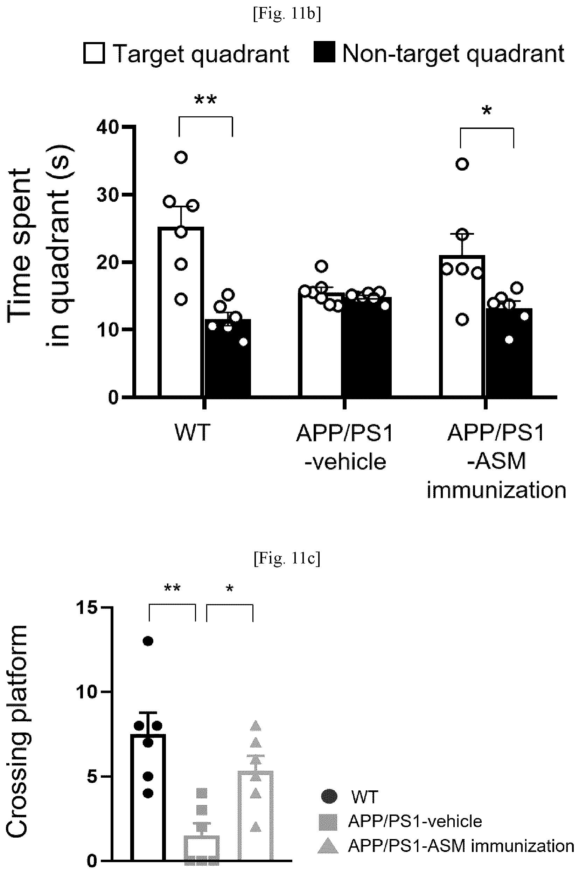

Fig. 11b shows the time spent on the target platform on the 11th day of the test.
Fig. 11c shows the number of entries into the target area of the platform on the 11th day of the test.

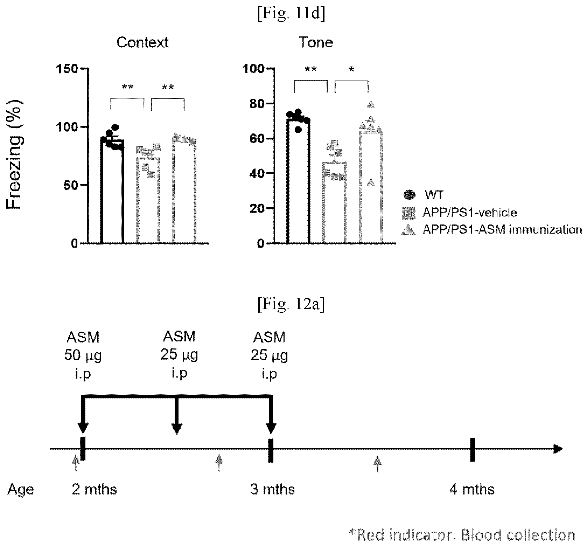

Fig. 11d shows the results of evaluating context and tone memory through the fear conditioning test in wild-type mice (n=6), Alzheimer's animal models injected with vehicle (n=6), and Alzheimer's animal models injected with ASM protein (n=6).

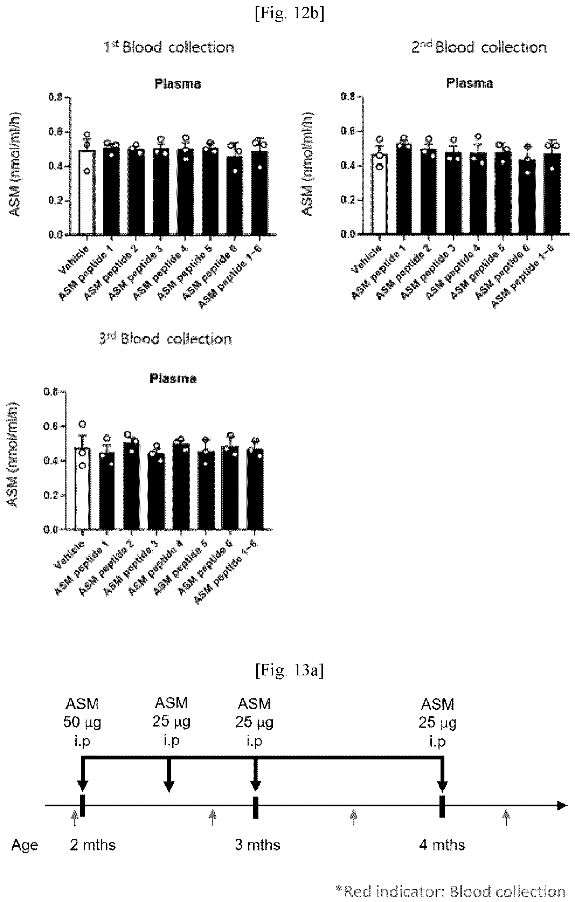

Figs. 12a and 12b are results confirming that injecting ASM protein fragments into wild-type mice does not suppress ASM activity in the plasma.
Fig. 12a is an experimental outline of injecting ASM protein fragments into wild-type mice.
Fig. 12b shows the results of measuring ASM activity in the plasma after injecting ASM protein fragments into wild-type mice (n=3).

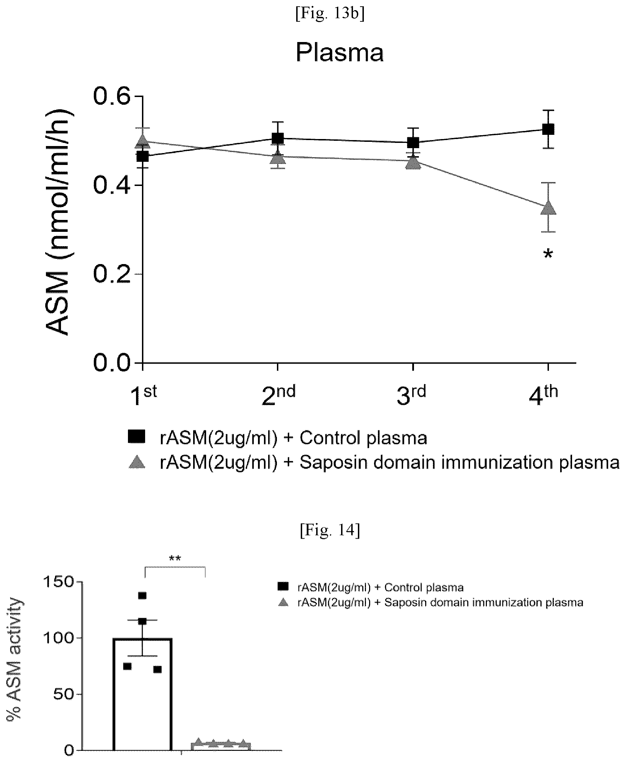

Figs. 13a and 13b are results confirming that injecting the saposin domain, a part of the ASM protein, into wild-type mice suppresses ASM activity in the plasma.
Fig. 13a is an experimental outline of injecting the saposin domain, a part of the ASM protein, into wild-type mice.
Fig. 13b shows the results of measuring ASM activity in the plasma after injecting the saposin domain, a part of the ASM protein, into wild-type mice (n=4).
Fig. 14 shows the results confirming that the plasma of wild-type mice injected with the saposin domain suppresses ASM protein activity (n=4).

### [Detailed Description of the Invention]

Hereinafter, the present invention will be described in detail with reference to the following examples. However, the following examples are merely illustrative of the present invention, and the present invention is not limited thereto.

### Experimental Methods

### 1. Recruitment of Alzheimer's Patients and Approval of Clinical Protocol

Blood was collected from Alzheimer's patients who met the diagnostic criteria for Alzheimer's disease based on normal or Amyloid PET analysis. This was conducted with approval based on the Hanyang University Hospital IRB research application number (HYUH 2016-12-029-003).

### 2. Mice

Approval for mouse experiments was obtained from the IACUC (Kyungpook National University Institutional Animal Care and Use Committee). Transgenic mouse lines overexpressing APPswe (hAPP695swe) or PS1 (presenilin-1M146V) were used based on C57BL/6 mice (Charles River, UK) [hereinafter, APP mice: mice overexpressing APPswe, PS1 mice: mice overexpressing presenilin-1M146V; GlaxoSmithKline]. APP/PS1 mice are an animal model in which beta-amyloid accumulation, a major brain lesion of Alzheimer's, and memory impairment become prominent from 7 months of age (Brain Res. 1017, 130-136 (2004)). To confirm the therapeutic effect of active immunization using ASM protein (recombinant human ASM, Gene script), ASM protein fragment (human ASM peptide, Gene script), and ASM saposin domain (Gene script) injection, 50 µg/100 µl of ASM protein, ASM protein fragment, ASM saposin domain, and 100 µl of PBS were mixed with 100 µl of complete Freund's adjuvant (Sigma-Aldrich, F5881) and intraperitoneally injected into 6-month-old APP/PS1 mice. The second and third injections were performed by mixing 25 µg/50 µl of ASM protein or 50 µl of PBS with 500 µl of incomplete Freund's adjuvant (Sigma-Aldrich, F5506) and intraperitoneally injecting them 2 weeks and 4 weeks after the initial injection, respectively, with the final injection conducted 4 weeks after the third injection. Two weeks after the final injection, behavioral analysis was conducted, and brain tissue samples were collected after the behavioral analysis (Fig. 2a). Blood was extracted a total of 5 times through orbital blood collection before PBS or ASM protein administration and after the first, second, third, and final administrations.

### 3. Measurement of ASM Activity

The concentration level of ASM was measured as follows. Specifically, 3 µl of mouse plasma sample or brain tissue was mixed with ASM activity buffer and incubated at 37°C. After adding 114 µl of ethanol to terminate the hydrolysis reaction, the mixture was centrifuged. 30 µl of the supernatant was transferred to a glass vial, and 5 µl was applied to the UPLC system. The ASM concentration level was quantified by comparing it with Bodipy (aminoacetaldehyde) bound to sphingomyelin and ceramide. The extraction and quantification of sphingomyelin and ceramide were performed by extracting lipids from known method samples, resuspending the dried lipid extract in 25 µl of 0.2% Igepal CA-630 (Sigma-Aldrich), and quantifying the concentration levels of each lipid using the UPLC system.

For the experiment measuring the inhibitory effect of ASM activity in mouse plasma, ASM activity was measured in 3 µl of a sample prepared by mixing 1.5 µl of mouse plasma and 1.5 µl of 4 µg/ml ASM protein (recombinant human ASM, Gene script) using the same method as above.

### 4. Flow Cytometry

To analyze Th17 and regulatory T (Treg) cells in blood, red blood cells were removed using histopaque (Sigma-Aldrich, 10771) solution, and lymphocytes were obtained. For monocyte and macrophage analysis, NH₄Cl (STEMCELL Technologies) solution was added to the blood to lyse red blood cells, and blood cells were obtained. To analyze immune cells in brain tissue, brain tissue extracted from mice was reacted with a solution containing collagenase P (Roche), dispase II (Roche), DNase I (Roche), and collagenase A (Roche) at 37°C for 1 hour, and then myelin was removed using a 25% BSA solution to obtain single cells. Th17 cells from lymphocytes obtained from blood and single cells obtained from brain tissue were stained using anti-CD4 FITC (BD Bioscience, 553047) and mouse anti-IL17 PerCp Cy5.5 (BD Bioscience, 553142) antibodies. Treg cells were stained using anti-CD4 FITC (BD Bioscience, 553047), anti-CD25 PE (eBioscience, 12-0251-82), and anti-FoxP3 APC (eBioscience, 17-5773-82) antibodies. Monocytes from blood cells obtained from blood were stained using APC-CD11b (BD, 553312), PE-CD115 (eBioscience, 12-1152-82), and FITC-Ly6C (BD, 553104) antibodies. Macrophages were stained using Biotin-Lineage (Milteny, 130-090-858), PE-CD11b (BD, 557397), APC-F480 (eBioscience, 17-4801-82), FITC-Ly6C (BD, 553104), and Pacific blue-streptavidin (Thermo, S11222) antibodies. Monocytes from single cells isolated from brain tissue were stained using Percp-cy5.5-CD45 (BD, 550994), PE-CD11b (BD, 557397), FITC-Ly6C (BD, 553104), and APC-cy7-Gr1 (BD, 557661) antibodies. Macrophages were stained using APC-cy7-CD45 (BD, 557659), Pecy5-CD11b (Tonbo, 55-011-U100), FITC-Ly6G (BD, 551460), APC-F480 (eBioscience, 17-4801-82), PE-MHCII (BD, 557000), and Pecy7-CD206 (eBioscience, 25-2061-82) antibodies. Data were collected using the Attune NxT flow cytometer (Thermo Fisher) and analyzed using FlowJo software (Tress Star).

### 5. Immunofluorescence

After fixing the cortex and hippocampus of mice, they were incubated with 0.5% thioflavin S (Sigma-Aldrich), anti-GFAP (rabbit, 1:500, DAKO), and anti-Iba-1 (rabbit, 1:500, WAKO). The regions were analyzed using a laser scanning confocal microscope equipped with Fluoview SV1000 imaging software (Olympus FV1000, Japan) or an Olympus BX51 microscope. The percentage of the stained area relative to the total tissue area was quantified and analyzed using Metamorph software (Molecular Devices).

### 6. Abeta40, Abeta42 ELISA

After extracting proteins from the cortex and hippocampus of mice, the amounts of Abeta40 (Invitrogen, KHB3481) and Abeta42 (Invitrogen, KHB3441) were measured using ELISA.

### 7. Western Blot

The expression of the following genes was analyzed using Western blotting. Specifically, antibodies against Zo1 (Invitrogen, 40-2200), Claudin5 (Invitrogen, 35-2500), Occludin (Invitrogen, 33-1500), Fibrin (Dako, A008002), Thrombin (Santa Cruz, sc-23355), synaptophysin (Abcam, ab32127), synapsin1 (Synaptic Systems, 106 103), PSD95 (Millipore, MAB1596), MAP2 (Abcam, ab5392), and β-actin (Santa Cruz) were used, and densitometric quantification was performed using ImageJ software (US National Institutes of Health).

### 8. Behavioral Experiments

To confirm the potential effects on learning and memory, the MWM (Morris water maze) experiment was conducted. MWM involved training mice on tasks 4 times a day for 10 days, followed by a probe trial on the 11th day after removing the platform.

For fear conditioning, on the first day, mice were placed in a conditioning chamber and given auditory stimuli (10 kHz, 70 dB) and electric stimuli (0.3 mA, 1s). On the second day, memory for the space was tested in the same conditioning chamber without stimuli, and on the third day, memory for fear was tested in a different conditioning chamber with only auditory stimuli.

### 9. Statistical Analysis

Student's T-test was performed for comparisons between two groups, while Tukey's HSD test and repeated measures analysis of variance were performed for comparisons among multiple groups using the SAS statistical package (release 9.1; SAS Institute Inc., Cary, NC). **p*<0.05, ***p*<0.01, ****p*<0.001, *****p*<0.0001 were considered significant.

### Experimental Results

### 1. Confirmation of ASM Activity in Plasma of Alzheimer's Patients and Animal Models

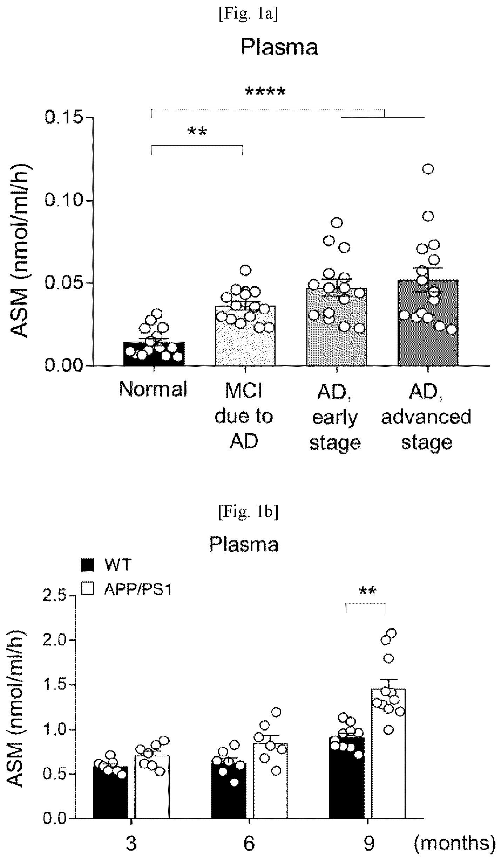

Before confirming the therapeutic effect of ASM protein injection for Alzheimer's, ASM activity was measured in the plasma of Alzheimer's patients at different disease stages and in Alzheimer's animal models at different ages. The results showed that ASM activity in the plasma of Alzheimer's patients increased at all disease stages compared to normal individuals, with higher ASM activity observed in patients with MCI (mild cognitive impairment) due to AD, early AD, and advanced AD (Fig. 1a). APP/PS1 mice, a representative Alzheimer's animal model, exhibit prominent brain lesions and memory impairment after 7 months of age (Brain Res. 1017, 130-136 (2004)). Measurement of ASM activity in the plasma of WT and APP/PS1 mice at 3, 6, and 9 months of age revealed no significant difference in ASM activity between WT and APP/PS1 mice at 3 and 6 months of age, but increased ASM activity was observed in the plasma of 9-month-old APP/PS1 mice (Fig. 1b).

### 2. Confirmation of Changes in ASM Activity in Alzheimer's Animal Models Administered with ASM Protein

To verify whether ASM protein administration is effective in preventing Alzheimer's lesions in 6-month-old APP/PS1 mice, an age before the increase in plasma ASM activity, 50 µg of ASM protein was intraperitoneally injected into 6-month-old APP/PS1 mice, followed by two additional injections of 25 µg of ASM protein at 2-week intervals. Finally, 25 µg of ASM protein was administered 4 weeks after the third injection (Fig. 2a). Plasma was collected before the initial injection and at each sampling point after the injections to measure changes in ASM activity.

The results confirmed that ASM activity in the plasma decreased in APP/PS1 mice administered with ASM protein (Fig. 2b).

### 3. Confirmation of Changes in Immune Cell Composition in Alzheimer's Animal Models Administered with ASM Protein

In Alzheimer's animal models, it is known that excessive immune cell-induced inflammatory responses in the blood and brain can affect Alzheimer's brain lesions. Specifically, it is known in the art that Th17 cells, one of the T helper (Th) cells that increase inflammatory responses, as well as monocytes and macrophages, are increased in Alzheimer's patients, while regulatory T (Treg) cells, which suppress inflammatory responses, are decreased. Based on this, it was confirmed whether the specifically decreased ASM activity in the plasma due to ASM antibodies generated by ASM protein administration affects the altered immune cell composition in Alzheimer's animal models.

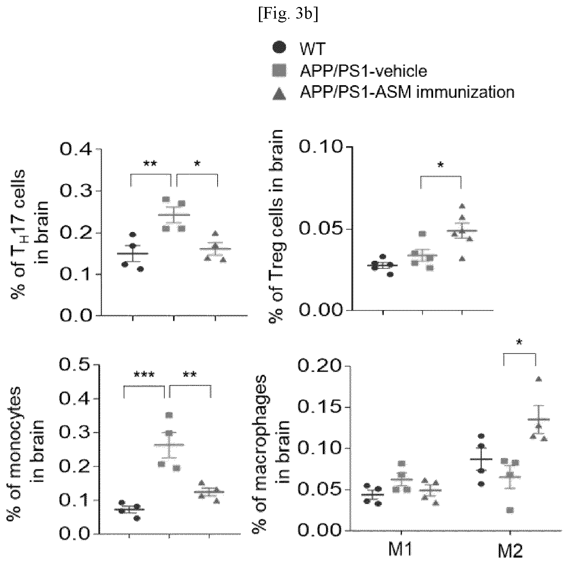

The results showed that Th17 cells, Ly6Chi monocytes (pro-inflammatory monocytes), and macrophages, which were increased in the blood of APP/PS1 mice compared to WT mice, did not increase due to ASM protein administration, and Treg cells and Ly6Clow (antiinflammatory monocytes), which were decreased, did not decrease (Fig. 3a). Additionally, in the brain, Th17 cells, monocytes, and macrophages, which were increased, did not increase due to ASM protein administration, while Treg cells increased (Fig. 3b). In other words, the inhibition of ASM activity in the plasma due to ASM protein administration was found to prevent abnormal immune cell composition changes in the blood and brain of Alzheimer's animal models.

### 4. Confirmation of Amyloid-β Deposition in Alzheimer's Animal Models Administered with ASM Protein

To confirm whether the inhibition of ASM activity in the plasma due to ASM protein injection is effective in preventing Alzheimer's lesions, the cortex and hippocampus of mice were stained with thioflavin S (ThioS) according to a known method to confirm fibrillar amyloid-β deposition. Additionally, amyloid-β deposition was confirmed by conducting ELISA for Aβ40 and Aβ42.

The experimental results showed that fibrillar Aβ deposition (Fig. 4a) and Aβ40 and Aβ42 (Fig. 4b) deposition in the cortex and hippocampus of APP/PS1 mice injected with ASM protein were significantly lower compared to APP/PS1 mice injected with vehicle. In other words, the inhibition of ASM activity in the plasma due to ASM protein injection was confirmed to prevent excessive amyloid-β deposition in the Alzheimer's brain environment.

### 5. Confirmation of Changes in Neuroinflammation in Alzheimer's Animal Models Administered with ASM Protein

To confirm the effect of the inhibition of ASM activity in the plasma due to ASM protein injection on neuroinflammation in Alzheimer's animal models, the inventors observed changes in microglia and astrocytes in the brain. Compared to APP/PS1 mice injected with vehicle, the activity of microglia (Iba-1) and astrocytes (GFAP) in the cortex and hippocampus of APP/PS1 mice administered with ASM protein was significantly reduced (Figs. 5a and 5b). Therefore, the inhibition of ASM activity in the plasma due to ASM protein injection was confirmed to prevent the increase in neuroinflammatory responses in the Alzheimer's brain environment.

### 6. Confirmation of Tight Junction Damage and Permeability Changes in the Blood-Brain Barrier of Alzheimer's Animal Models Administered with ASM Protein

To confirm the effect of the inhibition of ASM activity in the plasma due to ASM protein injection on blood-brain barrier (BBB) damage in Alzheimer's animal models, changes in BBB tight junction proteins and plasma proteins were observed in brain tissue.

The experimental results showed that the decrease in BBB tight junction proteins Zo1, claudin5, and occludin in the cortex and hippocampus of APP/PS1 mice injected with vehicle compared to WT mice was not observed in APP/PS1 mice administered with ASM protein (Fig. 6a). Additionally, the expression of plasma proteins fibrin and thrombin in the cortex and hippocampus of APP/PS1 mice administered with ASM protein did not increase compared to APP/PS1 mice injected with vehicle (Fig. 6b). These results indicate that the inhibition of ASM activity in the plasma due to ASM protein injection can prevent BBB damage and leakage in the Alzheimer's brain environment.

### 7. Confirmation of Changes in Synaptic Damage in Alzheimer's Animal Models Administered with ASM Protein

To confirm the effect of the inhibition of ASM activity in the plasma due to ASM protein injection on synaptic damage in Alzheimer's animal models, changes in synaptic proteins were observed in brain tissue.

The experimental results showed that the decrease in synaptic proteins synaptophysin, synapsin1, PSD95, and MAP2 in the cortex and hippocampus of APP/PS1 mice injected with vehicle compared to WT mice was not observed in APP/PS1 mice administered with ASM protein (Fig. 7). These results indicate that the inhibition of ASM activity in the plasma due to ASM protein injection can prevent synaptic damage in the Alzheimer's brain environment.

### 8. Confirmation of Memory Improvement in Alzheimer's Animal Models Administered with ASM Protein

To confirm whether the inhibition of ASM activity in the plasma due to ASM protein injection has a potential preventive effect on memory, MWM (Morris water maze) and fear conditioning tests were conducted.

As shown in Figs. 8a to 8d, Alzheimer's animal models injected with vehicle showed severe impairments in spatial memory, cognitive ability, and memory formation, whereas Alzheimer's animal models administered with ASM protein did not exhibit such impairments.

In summary, ASM protein injection in Alzheimer's animal models at an age before the increase in ASM activity and the appearance of Alzheimer's lesions was found to generate ASM antibodies in the plasma, specifically reducing ASM activity in the plasma. As a result, it was confirmed that abnormal immune cell composition changes in the blood and brain of Alzheimer's animal models, Aβ plaque deposition in brain tissue, neuroinflammation, BBB damage and increased permeability, synaptic damage, and reduced learning and memory abilities could be prevented. Therefore, the use of active immunization with ASM protein, i.e., as a vaccine, was found to be applicable as a preventive or therapeutic agent for degenerative neurological diseases such as Alzheimer's disease.

### 9. Confirmation of Amyloid-β Deposition in 12-Month-Old Alzheimer's Animal Models Administered with ASM Protein

To verify whether ASM protein administration is effective in improving Alzheimer's lesions through active immunization in 9-month-old APP/PS1 mice, an age after the increase in plasma ASM activity, 50 µg of ASM protein was intraperitoneally injected into 9-month-old APP/PS1 mice, followed by two additional injections of 25 µg of ASM protein at 2-week intervals. Finally, 25 µg of ASM protein was administered 4 weeks after the third injection (Fig. 9a).

The results confirmed that ASM activity in the plasma decreased in APP/PS1 mice administered with ASM protein (Fig. 9b). In other words, it was found that ASM protein administration could specifically inhibit ASM activity in the plasma of Alzheimer's animal models even at an age when ASM activity had increased.

Next, the cortex and hippocampus were stained with thioflavin S (ThioS) according to a known method to confirm fibrillar amyloid-β deposition.

The experimental results showed that fibrillar Aβ deposition in the cortex and hippocampus of APP/PS1 mice injected with ASM protein was significantly lower compared to APP/PS1 mice injected with vehicle (Fig. 10). In other words, the inhibition of ASM activity in the plasma due to ASM protein injection was confirmed to reduce excessive amyloid-β deposition in the Alzheimer's brain environment.

### 10. Confirmation of Memory Improvement in 12-Month-Old Alzheimer's Animal Models Administered with ASM Protein

To confirm whether the inhibition of ASM activity in the plasma due to ASM protein injection has a potential preventive effect on memory in 12-month-old Alzheimer's animal models, MWM (Morris water maze) and fear conditioning tests were conducted.

As shown in Figs. 11a to 11d, Alzheimer's animal models injected with vehicle showed severe impairments in spatial memory, cognitive ability, and memory formation, whereas Alzheimer's animal models administered with ASM protein did not exhibit such impairments.

In summary, ASM protein injection in Alzheimer's animal models not only at an age before the increase in ASM activity and the appearance of Alzheimer's lesions but also at an age after the increase in ASM activity and the appearance of Alzheimer's lesions was found to prevent the reduction in learning and memory abilities. Therefore, the use of active immunization with ASM protein, i.e., as a vaccine, was found to be applicable as a preventive or therapeutic agent for degenerative neurological diseases such as Alzheimer's disease.

### 11. Confirmation of Plasma ASM Activity in Wild-Type Mice Administered with ASM Protein Fragments

Six ASM protein fragments (SEQ ID NOs: 3 to 8) with high antigenicity were produced and injected, but it was confirmed that they did not inhibit plasma ASM activity (Figs. 12a and 12b).

### 12. Confirmation of Plasma ASM Activity in Wild-Type Mice Administered with the Saposin Domain of ASM Protein

The saposin domain of ASM protein plays an important role in ASM activity (Nature Com. 12196, (2016)). Therefore, it was confirmed that the saposin domain inhibited plasma ASM activity when injected (Figs. 13a and 13b).

### 13. Confirmation of Inhibition of ASM Protein Activity in Wild-Type Mice Administered with ASM Saposin Domain

It was confirmed that the activity of ASM protein (recombinant ASM) in the plasma of mice administered with ASM saposin domain was inhibited compared to the plasma of mice injected with vehicle (Fig. 14).

In summary, ASM protein injection was found to specifically reduce ASM activity in the plasma. However, while ASM protein fragments could not reduce ASM activity in the plasma, the injection of ASM saposin domain was found to reduce ASM activity in the plasma.

### [Industrial Applicability]

The composition of the present disclosure comprising ASM protein or a fragment thereof as an active ingredient has high industrial applicability due to its excellent effect in preventing or treating neurodegenerative diseases or depression caused by increased ASM protein activity, by inhibiting the activity of ASM protein in the blood.

## Claims

1. A pharmaceutical composition for preventing or treating neurodegenerative diseases or depression comprising ASM (acid sphingomyelinase) protein or a fragment thereof; a vector comprising a polynucleotide encoding ASM (acid sphingomyelinase) protein or a fragment thereof; or a cell comprising the vector as an active ingredient.

2. The pharmaceutical composition of claim 1, wherein the ASM comprises the amino acid sequence of SEQ ID NO: 1.

3. The pharmaceutical composition of claim 1, wherein the fragment is a saposin domain of ASM.

4. The pharmaceutical composition of claim 3, wherein the saposin domain of ASM comprises the amino acid sequence of SEQ ID NO: 2.

5. The pharmaceutical composition of claim 1, wherein the vector is at least one selected from the group consisting of linear DNA, plasmid DNA and recombinant viral vectors.

6. The pharmaceutical composition of claim 1, wherein the cell is at least one selected from the group consisting of hematopoietic stem cells, dendritic cells, autologous tumor cells and established tumor cells.

7. The pharmaceutical composition of claim 1, wherein the neurodegenerative disease is selected from the group consisting of dementia, Alzheimer's disease, Parkinson's disease, Huntington's disease, mild cognitive impairment, cerebral amyloid angiopathy, Down syndrome, amyloid stroke, systemic amyloidosis, Dutch-type amyloidosis, Niemann-Pick disease, Pick's disease, multiple sclerosis, Creutzfeldt-Jakob disease, amyotrophic lateral sclerosis, spinocerebellar atrophy, Tourette's syndrome, Friedrich's Ataxia, Machado-Joseph's disease, Lewy body dementia, dystonia, progressive supranuclear palsy and frontotemporal dementia.

8. A food composition for preventing or improving neurodegenerative diseases or depression comprising ASM (acid sphingomyelinase) protein or a fragment thereof; a vector comprising a polynucleotide encoding ASM (acid sphingomyelinase) protein or a fragment thereof; or a cell comprising the vector as an active ingredient.

9. Use of ASM (acid sphingomyelinase) protein or a fragment thereof; a vector comprising a polynucleotide encoding ASM (acid sphingomyelinase) protein or a fragment thereof; or a cell comprising the vector for preparing a composition for treating neurodegenerative diseases or depression.

10. The use of claim 9, wherein the ASM comprises the amino acid sequence of SEQ ID NO: 1.

11. The use of claim 9, wherein the neurodegenerative disease is selected from the group consisting of dementia, Alzheimer's disease, Parkinson's disease, Huntington's disease, mild cognitive impairment, cerebral amyloid angiopathy, Down syndrome, amyloid stroke, systemic amyloidosis, Dutch-type amyloidosis, Niemann-Pick disease, Pick's disease, multiple sclerosis, Creutzfeldt-Jakob disease, amyotrophic lateral sclerosis, spinocerebellar atrophy, Tourette's syndrome, Friedrich's Ataxia, Machado-Joseph's disease, Lewy body dementia, dystonia, progressive supranuclear palsy and frontotemporal dementia.

12. A method for treating neurodegenerative diseases or depression comprising administering to a subject in need thereof a composition comprising ASM (acid sphingomyelinase) protein or a fragment thereof; a vector comprising a polynucleotide encoding ASM (acid sphingomyelinase) protein or a fragment thereof; or a cell comprising the vector as an active ingredient.

13. The treatment method of claim 12, wherein the ASM comprises the amino acid sequence of SEQ ID NO: 1.

14. The treatment method of claim 12, wherein the neurodegenerative disease is selected from the group consisting of dementia, Alzheimer's disease, Parkinson's disease, Huntington's disease, mild cognitive impairment, cerebral amyloid angiopathy, Down syndrome, amyloid stroke, systemic amyloidosis, Dutch-type amyloidosis, Niemann-Pick disease, Pick's disease, multiple sclerosis, Creutzfeldt-Jakob disease, amyotrophic lateral sclerosis, spinocerebellar atrophy, Tourette's syndrome, Friedrich's Ataxia, Machado-Joseph's disease, Lewy body dementia, dystonia, progressive supranuclear palsy and frontotemporal dementia.
